# EUROPÄISCHE PATENTSCHRIFT

(11) **EP 1 628 594 B1**
(45) Veröffentlichungstag und Bekanntmachung des Hinweises auf die Patenterteilung: **08.04.2009**
(21) Anmeldenummer: 04739485.3
(22) Anmeldetag: 01.06.2004
(51) Int. Cl.: A61F 2/04, A61F 2/06

(54) **VERBINDUNGSSYSTEM ZUR VERBINDUNG EINES STENTS MIT EINEM RADIOOPAKEN MARKER SOWIE VERFAHREN ZUR HERSTELLUNG EINER VERBINDUNG ZWISCHEN EINEM STENT UND ZWEI ODER MEHREREN RADIOOPAKEN MARKERN**
CONNECTION SYSTEM FOR CONNECTING A STENT TO A RADIO-OPAQUE MARKER AND METHOD FOR GENERATION OF A CONNECTION BETWEEN A STENT AND TWO OR MORE RADIO-OPAQUE MARKERS
SYSTEME DE LIAISON SERVANT A RELIER UN STENT A UN MARQUEUR RADIO-OPAQUE ET PROCEDE DE PRODUCTION D'UNE LIAISON ENTRE UN STENT ET AU MOINS DEUX MARQUEURS RADIO-OPAQUES

(30) Priorität: 02.06.2003 DE 10325678
(43) Veröffentlichungstag der Anmeldung: 01.03.2006
(73) Patentinhaber: Biotronik GmbH & Co. KG, 12359 Berlin (DE)
(72) Erfinder: LOOTZ, Daniel, 18119 Rostock (DE); MOMMA, Carsten, 18055 Rostock (DE); BECHER, Bärbel, 18057 Rostock (DE); KIEKBUSCH, Martin, 18437 Stralsund (DE); MÜLLER, Heinz, 91052 Erlangen (DE)
(74) Vertreter: Lindner-Vogt, Karin L.
(86) Internationale Anmeldenummer: PCT/EP2004/005883
(87) Internationale Veröffentlichungsnummer: WO 2004/105642

(56) Entgegenhaltungen:
- EP-A- 1 212 991
- WO-A-02/15820
- US-A1- 2002 193 867
- US-A1- 2003 040 772

## Beschreibung

Die Erfindung betrifft ein Verbindungssystem zur Verbindung eines Stents mit einem radioopaken Marker sowie ein dazugehöriges Verfahren zur Herstellung einer Verbindung zwischen einem Stent und zwei oder mehrerer radioopaken Markern.

Stents, insbesondere für den koronaren Einsatz, werden in der Regel aus Metallen oder Metalllegierungen, z. B. rostfreiem 316L-Stahl oder Nitinol, aber auch speziellen Polymerwerkstoffen geformt. Die eingesetzten Materialien haben allerdings den Nachteil, dass sie gar nicht oder nur schwer radiologisch erfassbar sind. Die Röntgentechnik stellt aber das bei weitem leistungsfähigste Instrument zur Überwachung der Implantation, der relativen Lage und des Expansionszustands des Stents dar.

Zur Abhilfe ist es u.a. bekannt den Stent mit radioopaken Materialien zu beschichten. Dazu zählen insbesondere Metalle wie Platin, Palladium, Silber, Gold, Lanthanoide und deren Legierungen. Es hat sich allerdings gezeigt, dass sich eine indifferente Beschichtung über Teile oder gar die gesamte Stentoberfläche negativ auf die mechanischen Eigenschaften des Grundgerüsts des Stents auswirkt. Insbesondere beim Einsatz selbstexpandierender Stents wird eine Expansion im gewünschten Sinne behindert und kann allenfalls durch zusätzliche konstruktive Maßnahmen am Stentdesign kompensiert werden.

Aus der US 6,022,374 ist bekannt, den radioopaken Marker nicht direkt auf das Grundgerüst, sondern auf einen hiervon isolierten Bereich, der nicht bei der Expansion des Stents hinderlich ist, aufzubringen. Dazu ist ein Rahmenelement vorgesehen, das auf diesen speziellen Abschnitt des Grundgerüsts aufgesetzt wird und eine kreisförmige Öffnung mit nach innen gerichteten Spitzen umfasst. In diese Öffnung wird der Marker eingesetzt.

Das Dokument WO 02/15820 beschreibt ein Verbindungssystem gemäss dem Oberbegriff des Anspruchs 1, bzw. Verfahren gemäss dem Oberbegriff des Anspruchs 13.

Aufgabe der vorliegenden Erfindung ist es, ein Verbindungssystem bereitzustellen, das eine Verbindung des Stents mit einem radioopaken Marker ohne Verschlechterung der mechanischen Eigenschaften des Stents erlaubt und das mit möglichst geringem konstruktivem Aufwand eine für die Sondierung und Implantation des Stents ausreichende Haltekraft bereitstellt.

Diese Aufgabe wird durch das Verbindungssystem zur Verbindung eines Stents mit einem radioopaken Marker mit dem Merkmal des Anspruchs 1 gelöst.

Der erfindungsgemäße Stent zeichnet sich dadurch aus, dass das Verbindungssystem zumindest eine Spannverbindung aus einem Spannelement und einem Klemmelement umfasst. Es hat sich überraschenderweise gezeigt, dass die an sich einfachen konstruktiven Maßnahmen zu Realisation des erfindungsgemäßen Verbindungssystems sowohl eine zumindest während der Sondierung und Implantation des Stent ausreichende Haltekraft für den Marker zur Verfügung stellen, als auch keine oder nur vernachlässigbar geringe Einflüsse auf die mechanischen Eigenschaften des Stents haben.

Das erfindungsgemäße Verbindungssystem hat sich als besonders vorteilhaft beim Einsatz selbstexpandierender Stents erwiesen. Gerade hier müssen die konstruktiven Maßnahmen zur Anbindung eines Markers möglichst geringen oder gar keinen Einfluss auf die Mechanik des Grundgerüst haben. Hierbei muss insbesondere beachtet werden, dass bei Etablierung eines Verbindungssystems möglichst geringe thermische und/oder mechanische Einflüsse auf das Grundgerüst ausgeübt werden, da ansonsten die Eigenschaft des das Grundgerüst bildenden Werkstoffs als Gedächtnismaterial agieren zu können, unerwünscht beeinflusst oder gar verloren gehen kann. Mit Hilfe des erfindungsgemäßen Verbindungssystems kann dies sicher gestellt werden.

Das erfindungsgemäße Verbindungssystem kann auch bei biodegradierbaren Grundgerüsten des Stents, z.B. aus Magnesiumlegierungen, verwendet werden. Die Lage und Form des Verbindungssystems hat dabei keinen oder nur einen geringen Einfluss auf das Degradationsverhalten des Grundgerüstes, so dass ein gleichmäßiger Abbau im lebenden Organismus stattfindet.

Weiterhin ist bevorzugt, dass der Marker selbst als Spann- oder Klemmelement der Spannverbindung ausgebildet ist. Es ist demnach nicht notwendig durch vorgelagerte Bearbeitungsschritte Spann- oder Klemmelemente aus einem anderen Material an den Marker anzuformen.

In einer weiteren Ausgestaltung des Erfindungsgedankens ist der Marker aus einem biokompatiblen Material geformt, wenn der Marker zum längerfristigen oder dauerhaften Verbleib im Körper des Patienten ausgelegt ist. Gerade bei biodegradierbaren Stents soll auf diese Weise mittel- und langfristigen Komplikationen infolge von Abstoßungsreaktionen des Körpers vorgebeugt werden. Insbesondere ist bevorzugt, wenn der Marker ganz oder in Teilen aus einem oder mehreren Metalle der Gruppe Ta, Nb, Zr, Hf, Mo, W, Au, Pt, Ir, seltene Erden oder deren Legierungen, insbesondere Ptlr, besteht. Die genannten Materialien zeichnen sich durch gute Verfügbarkeit, hohe Biokompatibilität und leichte Verarbeitbarkeit aus.

Ferner ist bevorzugt, dass das Spann- oder Klemmelement der Spannverbindung am Grundgerüst des Stents angeformt ist, d. h. nicht Bestandteil desselben ist. Hierdurch können sehr wirkungsvoll störende Einflüsse des Verbindungssystems auf den Übergang vom nicht-expandierten in den expandierten Zustand des Stents, als auch auf die mechanische Stabilität des Stents im nicht-expandierten und expandierten Zustand minimiert bzw. ausgeschlossen werden.

Bevorzugt ist weiterhin, das Spann- oder Klemmelement an beiden (proximalen) Enden des Stents anzuordnen. Hierdurch kann das Herstellungsverfahren vereinfacht werden, da die proximalen Enden des Stents leichter zugänglich sind. Zudem ist es vorteilhaft, das Verbindungssystem derart in das Grundgerüst zu integrieren, dass es nicht oder allenfalls in einem geringem Ausmaß in radialer Erstreckung über die Abmessungen der Umlaufwandung des Grundgerüsts ragt. Somit kann durch die spezielle Lage vermieden werden, dass die Spannverbindung aus der Ebene des Grundgerüsts herausragt und damit bei der Sondierung bzw. Implantation zu Gefäßverletzungen führen könnte.

Ein weiterer Aspekt der Erfindung betrifft ein Verfahren zur Herstellung einer Verbindung zwischen dem Stent und zwei oder mehr radioopaken Markern und zwar unter Verwendung eines Verbindungssystems mit den vorgenannten Merkmalen. Das Verfahren zeichnet sich dadurch aus, dass
(a) zwei oder mehr Marker über ein Positionierelement miteinander verbunden werden, so dass die Marker mit Ihren Spann- oder Klemmelementen auf die korrespondierenden Spann- oder Klemmelemente des Stents ausgerichtet sind,
(b) die Marker in einem Arbeitsschritt mit ihren Spann- oder Klemmelementen auf die korrespondierenden Spann- oder Klemmelemente des Stent gesetzt werden und
(c) anschließend die Verbindung zwischen dem Positionierelement und den Spann- oder Klemmelementen des Markers getrennt wird.

Durch die vorgenannte Verfahrensdurchführung können in kurzer Zeit mehrere Marker gleichzeitig oder nahezu gleichzeitig mit einem Stent verbunden werden. Durch die besondere Ausgestaltung mittels eines die einzelnen Marker verbindenden Positionierelementes kann eine hohe Positioniergenauigkeit erreicht werden, d. h. die Marker sind für radiologische Untersuchungsmethoden hinreichend genau zentriert und fixiert.

Unter dem Begriff "Spannelement" wird eine offene Rahmenkonstruktion verstanden, deren kurzfristige Auslenkung aus einer Ruhelage zur Ausbildung einer Rückstellkraft (Spannkraft) führt.

Unter dem Begriff "Klemmelement" wird ein Konstruktionselement verstanden, dass in seiner Formgebung und Abmessungen zur Aufnahme in das Spannelement angepasst ist.

Unter dem Begriff "Spannverbindung" wird eine Verbindung aus einem Spannelement und einem Klemmelement verstanden, bei der das Klemmelement nach Aufnahme in das Spannelement kraftschlüssig in Kontakt mit dem Spannelement gehalten wird.

Unter dem Begriff "Grundgerüst" werden alle konstruktiven Teilelemente des Stents verstanden, die beim Übergang vom nicht-expandierten in den expandierten Zustand des Stents mechanischen Belastungen ausgesetzt sind und die im nicht-expandierten und expandierten Zustand zur mechanischen Stabilität des Stents beitragen.

Die Erfindung wird nachfolgend anhand von Ausführungsbeispielen und den dazugehörigen Zeichnungen näher erläutert. Es zeigen:
- Fig. 1: ein proximales Abschnittsende eines Stents mit einem erfindungsgemäßen Verbindungssystem;
- Fig. 2a-c: einen vergrößerten Ausschnitt aus dem Stent gemäß Fig. 1 im Bereich des erfindungsgemäßen Verbindungssystems vor und nach Aufnahme zweier verschiedenartig dimensionierter Marker;
- Fig. 3a-c: drei alternative Ausführungsbeispiele für Spannelemente und
- Fig. 4: eine Prinzipdarstellung zur Erläuterung des erfindungsgemäßen Verfahrens zur Herstellung einer Verbindung zwischen einem Stent und zwei oder mehr radioopaken Markern.

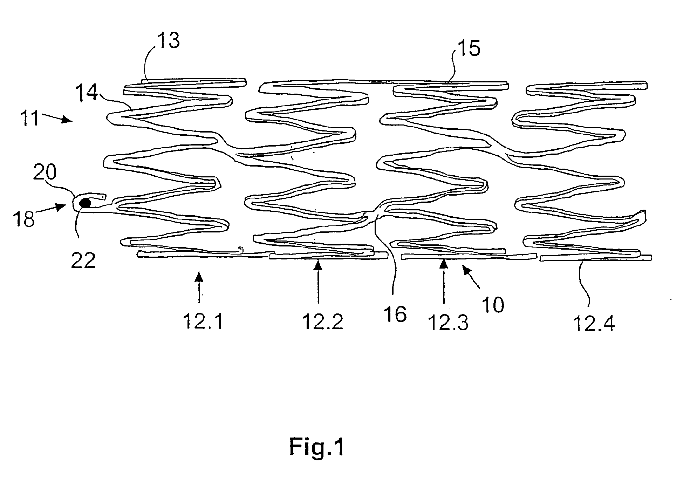

Die Fig. 1 zeigt schematisch eines Teilabschnitt eines Stents 10 im Bereich seines proximalen Endes 11 und zwar in einer Draufsicht auf eine Abwicklung seiner rohrförmig verlaufenden Umlaufwandung 13. Ein Grundgerüst 15 des Stents 10 setzt sich aus einer Vielzahl von Segmenten 12.1, 12.2, 12.3, 12.4 zusammen, die wiederum aus mäanderförmig in Umfangrichtung verlaufenden Stegen 14 bestehen, wobei die einzelnen Segmente 12.1, 12.2, 12.3, 12.4 in ausgewählten Bereichen über Brücken 16 miteinander verbunden sind. Das aufgezeigte Stentdesign ist selbstverständlich nur beispielhaft zu verstehen. Es versteht sich von selbst, dass beliebige Variationen - wie sie aus dem Stand der Technik in großer Vielfalt bekannt sind - im Zusammenhang mit dem erfindungsgemäßen Verbindungssystem genutzt werden können.

Das Verbindungssystem 18 umfasst ein Spannelement 20 und ein korrespondierendes Klemmelement 22. Das Spannelement 20 ist endständig am proximalen Ende 11 des Stents 10 angeordnet. Im konkreten Fall ist das Klemmelement aus einem radioopaken Material geformt und stellt damit für sich den radioopaken Marker dar.

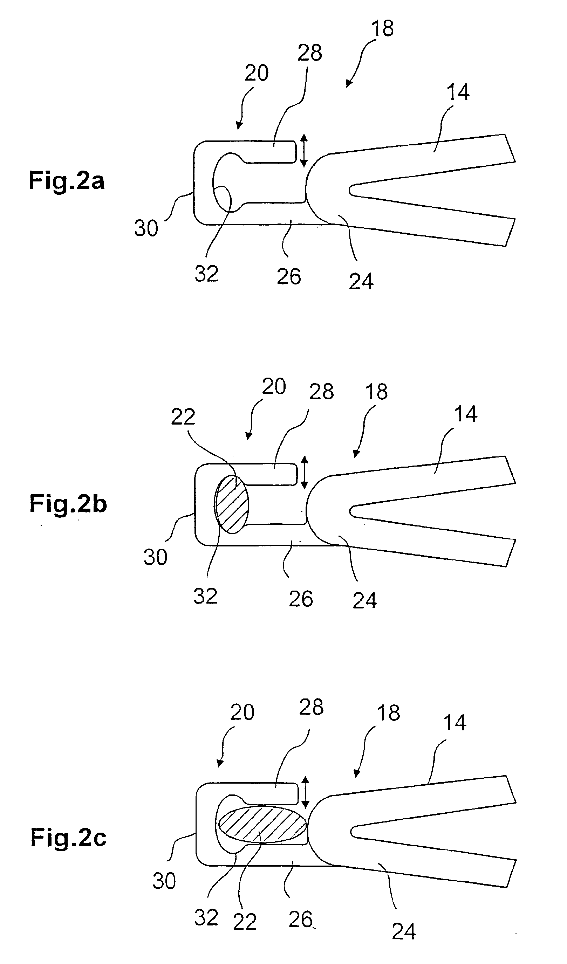

Den Fig. 2a, 2b und 2c, die einen vergrößerten Ausschnitt aus dem Stent 10 gemäß Fig. 1 im Bereich des Verbindungssystems 18 zeigen, sind nähre Details zu entnehmen. Das Spannelement 20 ist in einem endständigen Krümmungsbereich 24 eines Steges 14 an das Grundgerüst 15 des Stents 10 angeformt. Zur Anformung kann auf herkömmliche Verbindungstechniken zurückgegriffen werden, die jeweils individuell auf die gewählten Materialien des Stents 10 und des Klemmelements 20 abzustimmen sind. Eine Materialauswahl für das Spannelement 20 wird nur durch die folgenden Prämissen beschränkt:
- Das Material muss aufgrund seiner Verwendung biokompatibel sein.
- Das Material muss sich zur Realisation einer offenen Rahmenkonstruktion eignen, deren kurze Auslenkung eines Rahmenelementes aus der Ruhelage zur Ausbildung einer Rückstellkraft (Spannkraft) führt, ohne dass die Konstruktion bricht.

Denkbar ist auch, das Spannelement 20 aus einem biodegradierbaren Material zu formen, insbesondere dann, wenn der Stent 10 ebenfalls diese Eigenschaft aufweisen soll.

Wie in den Fig. 2a-2c ersichtlich ist das Spannelement 20 als ein klammerförmiges Element ausgebildet, dessen einer Arm 26 direkt im Krümmungsbereich 24 des Stents 10 angeformt ist. Ein zweiter, etwas kürzerer Arm 28 weist zwar in Richtung des Krümmungsbereiches 24, ist jedoch nicht mit diesem verbunden. Eine in Bezug auf eine Längsachse des Stents 10 radiale Erstreckung des Spannelementes 20 entspricht maximal den entsprechenden Abmessungen des Grundgerüst 15 des Stents 10, d. h. das Spannelement 20 ragt nicht über die von den einzelnen Stegen 14 der Umlaufwandung 13 hinaus. Auf diese Weise kann die Gefahr von etwaigen Gefäßverletzungen minimiert werden.

Das klammerförmige Spannelement 20 weist in seinem Ende 30 eine innere, kreisförmige Erweiterung 32 auf, die als Aufnahme für das Klemmelement 22 - hier dem radioopaker Marker - dient. Der Bereich 32 kann in seiner Form dem Klemmelement 22 angepasst werden, so dass das Klemmelement 22 form- und kraftschlüssig gehalten wird. Die Figur 2b und 2c zeigen zwei verschiedenartig dimensionierte Klemmelemente 22, die von dem Spannelement 20 aufgenommen werden können. Wie durch den Pfeil in der Fig. 2a angedeutet, zeigt der zweite Arm 28 ein elastisches Verhalten, wenn er aus seiner Ruhelage ausgelenkt wird. So wird vor oder während des Platzierens des Klemmelementes 22 der zweite Arm 28 vom ersten Arm 26 wegbewegt, fährt jedoch aufgrund seiner elastischen Eigenschaften mit einer bestimmten Rückstellkraft wieder seine ursprüngliche Lage ein. Diese Rückstellkraft kann zumindest in Teilen dazu genutzt werden das Klemmelement 22 nach der Aufnahme in der Erweiterung 32 zu halten.

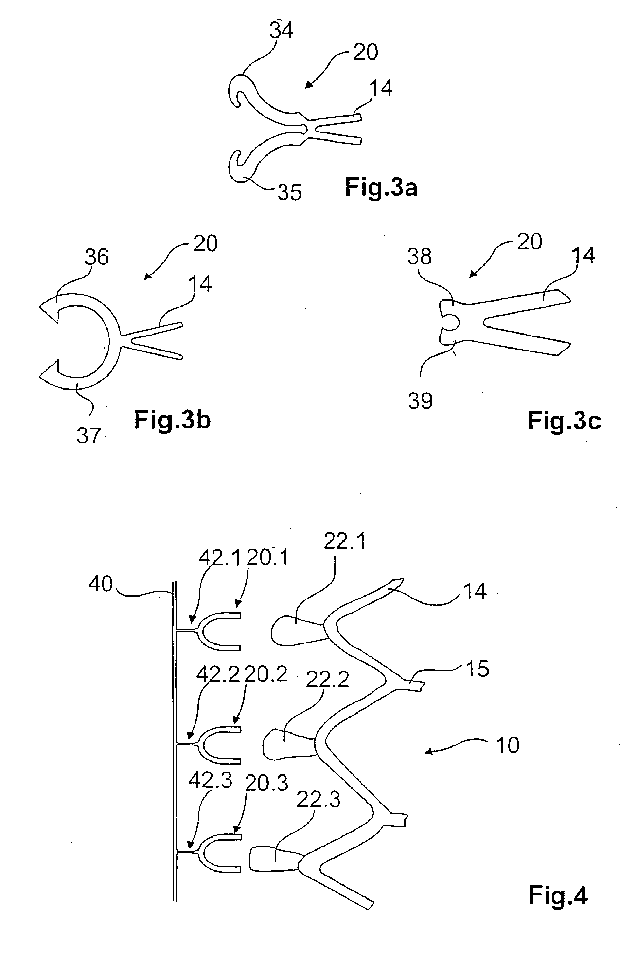

In den Figuren 3a bis 3c sind weitere, alternative Ausführungsformen des Spannelementes 20 skizziert. Fig. 3a zeigt ein Spannelement 20 mit zwei, eine trichterförmige Öffnung definierenden, hakenförmigen Armen 34, 35. Diese Ausführungsform des Spannelementes 20 eignet sich für Klemmelemente mit blättchen- oder bandförmiger Kontur. Fig. 3b offenbart ein Spannelement 20 mit zwei Armen 36, 37, die eine C-förmige Öffnung für ein Klemmelement bereitstellen. Das Klemmelement für ein solches Spannelement 20 ist vorzugsweise zylinderförmig oder halbzylinderförmig. Schließlich zeigt Fig. 3c ein Spannelement 20 mit zwei kurzen, eine u-förmige Öffnung definierenden Armen 38, 39. Diese Ausführungsform eignet sich insbesondere für schmalzylindrische Klemmelemente, wie kleine Drahtabschnitte und dergleichen.

Die eingesetzten Marker bestehen aus einem biokompatiblen Material. Sie können ganz oder in Teilen aus einem oder mehreren der Metalle der Gruppe Ta, Nb, Zr, Hf, Mo, W, Au, Pt, Ir, seltene Erden oder deren Legierungen, z. B. Ptlr bestehen. Zur besseren radiologischen Unterscheidbarkeit besitzen sie eine in zumindest eine Raumachsrichtung unterscheidbare Form, d. h. sie sind insbesondere nicht kugelsymmetrisch.

In den zuvor geschilderten Ausführungsbeispielen war das Klemmelement 22 jeweils der radioopake Marker selbst.

Fig. 4 zeigt eine Ausführungsform, in der der radioopake Marker als Spannelement 20.1, 20.2, 20.3 ausgebildet ist. In diesem Fall sind am Grundgerüst 15 des Stents 10 mehrere tropfenförmige Klemmelemente 22 angeformt. Für diesen speziellen Fall hat es sich als besonders nützlich erwiesen, wenn der radioopake Marker aus Tantal besteht, da dieser Werkstoff hoch röntgendicht und leicht verformbar ist sowie eine ausreichende Biokompatibilität besitzt. Im eben gleichem Sinne könnten Nb, Zr, Hf, Mo, W, Au, Pt, Ir, seltene Erden oder deren Legierungen eingesetzt werden.

Eine Verbindung zwischen dem Stent 10 und dem radioopaken Marker kann beispielsweise derart erfolgen, dass aus einem Bestückungsreservoir, das eine Vielzahl von Klemmelementen beinhaltet, mittels eines Positioniersystems jeweils ein Klemmelement in einem Spannelement platziert wird. Derartige Positioniersysteme sind dem Prinzip nach aus dem Stand der Technik bekannt und hochvariabel ausgestaltbar, so dass an dieser Stelle hierauf nicht näher eingegangen werden soll.

Sollen zwei oder mehr radioopake Marker gleichzeitig mit einem Stent verbunden werden, so kann wie in der Fig. 4, schematisch angedeutet, vorgegangen werden. Die insgesamt drei Spannelemente 20.1, 20.2, 20.3 aus einem radioopaken Material werden mit Hilfe eines Positionierelementes 14 gleichzeitig oder nahezu gleichzeitig auf den insgesamt drei Klemmelementen 22, die am Grundgerüst 11 des Stents 10 angeformt sind, platziert. Das Positionierelement 14 ist jeweils über einen kleinen Steg 42.1, 42.2, 42.3 mit den Spannelementen 20.1, 20.2, 20.3 verbunden. Nachdem die Spannelemente 20.1, 20.2, 20.3 auf die jeweils korrespondierenden Klemmelemente 22.1, 22.2, 22.3 gesetzt wurden, werden die Stege 42.1, 42.2, 42.3, z. B. mittels Laserschneidens, getrennt.

## Patentansprüche

1. Verbindungssystem (18) zur Verbindung eines Stents (10) mit einem radioopaken Marker, **dadurch gekennzeichnet, dass** das Verbindungssystem (18) zumindest eine Spannverbindung (18) aus einem Spannelement (20, 20.1, 20.2, 20.3) und einem Klemmelement (22, 22.1, 22.2, 22.3) umfasst.

2. Verbindungssystem nach Anspruch 1, **dadurch gekennzeichnet, dass** der Marker selbst als Spann- oder Klemmelement (20, 20.1, 20.2, 20.3, 22, 22.1, 22.2, 22.3) der Spannverbindung (18) ausgebildet ist.

3. Verbindungssystem nach den Ansprüchen 1 oder 2, **dadurch gekennzeichnet, dass** der Marker aus einem biokompatiblen Material geformt ist.

4. Verbindungssystem nach den Anspruch 3, **dadurch gekennzeichnet, dass** der Marker ganz oder in Teilen aus einem oder mehreren der Metalle der Gruppe Ta, Nb, Zr, Hf, Mo, W, Au, Pt, Ir, seltene Erden oder deren Legierungen besteht.

5. Verbindungssystem nach den Anspruch 4, **dadurch gekennzeichnet, dass** der Marker ganz oder in Teilen aus Ptlr besteht.

6. Verbindungssystem nach den Anspruch 1, **dadurch gekennzeichnet, dass** das Spann- oder Klemmelement (20, 20.1, 20.2, 20.3, 22, 22.1, 22.2, 22.3) der Spannverbindung (18) an einem Grundgerüst (15) des Stents (10) angeformt ist.

7. Verbindungssystem nach Anspruch 6, **dadurch gekennzeichnet, dass** das Verbindungssystem (18) derart in das Grundgerüst (15) integriert ist, dass es nicht oder allenfalls in einem geringem Ausmaß in radialer Erstreckung über die Abmessungen einer Umlaufwandung (13) des Grundgerüsts (15) ragt.

8. Verbindungssystem nach den Ansprüchen 6 oder 7, **dadurch gekennzeichnet, dass** das Spann- oder Klemmelement (22) am proximalen Ende des Stents (10) angeordnet ist.

9. Verbindungssystem nach Anspruch 6, **dadurch gekennzeichnet, dass** das Spann- oder Klemmelement (20, 20.1, 20.2, 20.3, 22, 22.1, 22.2, 22.3) aus einem biodegradierbaren Material geformt ist.

10. Verbindungssystem nach einem oder mehreren der vorhergehenden Ansprüche, **dadurch gekennzeichnet, dass** der Stent (10) selbstexpandierend ist.

11. Verbindungssystem nach einem oder mehreren der vorhergehenden Ansprüche, **dadurch gekennzeichnet, dass** der Stent (10) biodegradierbar ist.

12. Verbindungssystem nach Anspruch 11, **dadurch gekennzeichnet, dass** der Stent (10) ganz oder in Teilen aus einer biodegradierbaren Mg-Legierung geformt ist.

13. Verfahren zur Herstellung einer Verbindung zwischen einem Stent (10) und zwei oder mehr radioopaken Markern mittels eines Verbindungssystems (18) nach einem oder mehreren der Ansprüche 1 bis 13, **dadurch gekennzeichnet, dass**
(a) zwei oder mehr Marker über ein Positionierelement (40) miteinander verbunden werden, so dass die Marker mit Ihren Spann- oder Klemmelementen (20, 20.1, 20.2, 20.3, 22, 22.1, 22.2, 22.3) auf die korrespondierenden Spann- oder Klemmelemente (20, 20.1, 20.2, 20.3, 22, 22.1, 22.2, 22.3) des Stents (10) ausgerichtet sind,
(b) die Marker in einem Arbeitsschritt mit ihren Spann- oder Klemmelementen (20, 20.1, 20.2, 20.3, 22, 22.1, 22.2, 22.3) auf die korrespondierenden Spann- oder Klemmelemente (20, 20.1, 20.2, 20.3, 22, 22.1, 22.2, 22.3) des Stent (10) gesetzt werden und
(c) anschließend die Verbindung zwischen dem Positionierelement (40) und den Spann- oder Klemmelementen (20, 20.1, 20.2, 20.3, 22, 22.1, 22.2, 22.3) des Markers getrennt wird.

## Claims

1. A connecting system (18) for connecting a stent (10) to a radiopaque marker,
**characterized in that**
the connecting system (18) comprises at least one chucking connection (18) from one chucking element (20, 20.1, 20.2, 20.3) and one clamping element (22, 22.1, 22.2, 22.3).

2. The connecting system according to Claim 1,
**characterized in that**
the marker itself is designed as a chucking element or a clamping element (20, 20.1, 20.2, 20.3, 22, 22.1, 22.2, 22.3) of the chucking connection (18).

3. The connecting system according to Claims 1 or 2,
**characterized in that**
the marker is molded from a biocompatible material.

4. The connecting system according to Claim 3,
**characterized in that**
the marker consists entirely or in part of one or more of the metals from the group Ta, Nb, Zr, Hf, Mo, W, Au, Pt, Ir, rare earths or alloys thereof.

5. The connecting system according to Claim 4,
**characterized in that**
the marker consists entirely or in part of Ptlr.

6. The connecting system according to Claim 1,
**characterized in that**
the chucking element or the clamping element (20, 20.1, 20.2, 20.3, 22, 22.1, 22.2, 22.3) of the chucking connection (18) is integrally molded on a basic structure (15) of the stent (10).

7. The connecting system according to Claim 6,
**characterized in that**
the connecting system (18) is integrated into the basic structure (15) in such a way that it does not protrude at all or at least does so only to a slight extent in the radial direction beyond the dimensions of a circumferential wall (13) of the basic structure (15).

8. The connecting system according to Claims 6 or 7,
**characterized in that**
the chucking element or the clamping element (22) is arranged on the proximal end of the stent (10).

9. The connecting system according to Claim 6,
**characterized in that**
the chucking element or the clamping element (20, 20.1, 20.2, 20.3, 22, 22.1, 22.2, 22.3) is molded from a biodegradable material.

10. The connecting system according to any one or more of the preceding claims,
**characterized in that**
the stent (10) is self-expanding.

11. The connecting system according to any one or more of the preceding claims,
**characterized in that**
the stent (10) is biodegradable.

12. The connecting system according to Claim 6,
**characterized in that**
the stent (10) is molded entirely or in part from biodegradable Mg alloy.

13. The method for establishing a connection between a stent (10) and two or more radiopaque markers by means of a connecting system (18) according to any one or more of Claims 1 to 12,
**characterized in that**
(a) two or more markers are connected to each other by a positioning element (40), so that the markers are aligned with their chucking elements or clamping elements (20, 20.1, 20.2, 20.3, 22, 22.1, 22.2, 22.3) with the corresponding chucking elements or clamping elements (20, 20.1, 20.2, 20.3, 22, 22.1, 22.2, 22.3) of the stent (10),
(b) in one working step, the markers are placed with their chucking elements or clamping elements (20, 20.1, 20.2, 20.3, 22, 22.1, 22.2, 22.3) on the corresponding chucking elements or clamping elements (20, 20.1, 20.2, 20.3, 22, 22.1, 22.2, 22.3) of the stent (10), and
(c) then the connection between the positioning element (40) and the chucking elements or clamping elements (20, 20.1, 20.2, 20.3, 22, 22.1, 22.2, 22.3) of the marker is severed.

## Revendications

1. Système de liaison (18) pour la liaison d'un stent (10) avec un marqueur radio-opaque, **caractérisé en ce que** le système de liaison (18) comporte au moins une liaison de serrage (18) constitué d'un élément de serrage (20, 20.1, 20.2, 20.3) et d'un élément de blocage (22, 22.1, 22.2, 22.3).

2. Système de liaison selon la revendication 1, **caractérisé en ce que** le marqueur même est conçu comme élément de serrage ou de blocage (20, 20.1, 20.2, 20.3, 22, 22.1, 22.2, 22.3) de la liaison de serrage (18).

3. Système de liaison selon la revendication 1 ou 2, **caractérisé en ce que** le marqueur est formé à base d'un matériau biocompatible.

4. Système de liaison selon la revendication 3, **caractérisé en ce que** le marqueur est constitué tout ou en partie de l'un ou de plusieurs des métaux du groupe Ta, Nb, Zr, Hf, Mo, W, Au, Pt, Ir, terres rares ou de leurs alliages.

5. Système de liaison selon la revendication 4, **caractérisé en ce que** le marqueur est constitué tout ou en partie de Ptlr.

6. Système de liaison selon la revendication 1, **caractérisé en ce que** l'élément de serrage ou de blocage (20, 20.1, 20.2, 20.3, 22, 22.1, 22.2, 22.3) de la liaison de serrage (18) est formé sur une structure de base (15) du stent (10).

7. Système de liaison selon la revendication 6, **caractérisé en ce que** le système de liaison (18) est intégré dans la structure de base (15) de telle sorte qu'il ne dépasse pas ou tout du moins dans une faible proportion dans l'extension radiale des dimensions d'une paroi périphérique (13) de la structure de base (15).

8. Système de liaison selon la revendication 6 ou 7, **caractérisé en ce que** l'élément de serrage ou de blocage (22) est disposé sur l'extrémité proximale du stent (10).

9. Système de liaison selon la revendication 6, **caractérisé en ce que** l'élément de serrage ou de blocage (20, 20.1, 20.2, 20.3, 22, 22.1, 22.2, 22.3) est formé à base d'un matériau biodégradable.

10. Système de liaison selon l'une quelconque ou plusieurs des revendications précédentes, **caractérisé en ce que** le stent (10) est autoexpansible.

11. Système de liaison selon l'une quelconque ou plusieurs des revendications précédentes, **caractérisé en ce que** le stent (10) est biodégradable.

12. Système de liaison selon la revendication 11, **caractérisé en ce que** le stent (10) est formé tout ou en parties d'un alliage de Mg biodégradable.

13. Procédé pour fabriquer une liaison entre un stent (10) et deux ou plus de deux marqueurs radio-opaques au moyen d'un système de liaison (18) selon l'une quelconque ou plusieurs des revendications 1 à 12, **caractérisé en ce que**
(a) deux ou plus de deux marqueurs sont reliés les uns aux autres au moyen d'un élément de positionnement (40), de sorte que les marqueurs avec leurs éléments de serrage ou de blocage (20, 20.1, 20.2, 20.3, 22, 22.1, 22.2, 22.3) sont axés sur les éléments de serrage ou de blocage (20, 20.1, 20.2, 20.3, 22, 22.1, 22.2, 22.3) correspondants du stent (10),
(b) les marqueurs sont placés lors d'une étape de travail avec leurs éléments de serrage ou de blocage (20, 20.1, 20.2, 20.3, 22, 22.1, 22.2, 22.3) sur les éléments de serrage ou de blocage (20, 20.1, 20.2, 20.3, 22, 22.1, 22.2, 22.3) correspondants du stent (10),
(c) la liaison entre l'élément de positionnement (40) et les éléments de serrage ou de blocage (20, 20.1, 20.2, 20.3, 22, 22.1, 22.2, 22.3) du marqueur est séparée ensuite.
